(19) Europäisches Patentamt / European Patent Office / Office européen des brevets

(11) **EP 1 840 119 A2**

(12) **EUROPEAN PATENT APPLICATION**

(43) Date of publication:
**03.10.2007 Bulletin 2007/40**

(51) Int Cl.:
*C07C 319/20* (2006.01)   *C07C 323/58* (2006.01)

(21) Application number: **07104999.3**

(22) Date of filing: **27.03.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **27.03.2006 JP 2006084746**

(71) Applicant: **Sumitomo Chemical Company, Limited Tokyo 104-8260 (JP)**

(72) Inventors:
• **Fujita, Kazuo**
  **Ehime-ken Ehime (JP)**
• **Tokumasu, Yoshihisa**
  **Ehime-ken Ehime (JP)**

(74) Representative: **Vossius & Partner**
**Siebertstrasse 4**
**81675 München (DE)**

(54) **Process for producing methionine**

(57) Disclosed is a process for producing methionine, which comprises hydrolyzing 5-(β-methylmercaptoethyl)hydantoin using at least one potassium compound selected from potassium carbonate, potassium bicarbonate and potassium hydroxide, crystallizing the hydrolyzate under pressure of carbonic acid gas thereby separating and collecting methionine, and heat treating at least a portion of the resulting filtrate thereby recovering methionine dimer contained in the filtrate as methionine, the process being characterized by using, as a material of an equipment of a facility for recovery through a heat treatment, a ferritic stainless steel containing 17.0 to 32.0% by weight of Cr element of, 0.75 to 3.0% by weight of Mo element and 0.6% by weight or less of Ni element, or zirconium.

**EP 1 840 119 A2**

**Description**

The present invention relates to a process for producing methionine which is useful as a feed additive. More particularly, it relates to a process for stably producing methionine while preventing corrosion of a facility for recovering as methionine through a heat treatment of methionine dimer contained in the filtrate formed from crystallization for obtaining methionine.

There has been known a process which comprises hydrolyzing 5-(β-methylmercaptoethyl)hydantoin (hereinafter sometimes simply abbreviated to hydantoin) using at least one potassium compound selected from potassium carbonate, potassium bicarbonate and potassium hydroxide, crystallizing the hydrolyzate under pressure of carbonic acid gas thereby separating and collecting methionine, and heat treating at least a portion of the resulting filtrate thereby recovering methionine dimer contained in the filtrate as methionine (see U.S. Patent No. 5,945,563).

The heat treatment of this filtrate is commonly performed at a temperature of about 150 to 200°C. Since hydrolysis of methionine dimer is accelerated when the temperature increases, a stainless steel such as SUS316L provided by JIS G 4305 has a problem such as insufficient corrosion resistance.
Also, a duplex stainless steel such as SUS329J4L exhibits better corrosion resistance than that of SUS316L but is not complete, and thus it is required to develop a material having more excellent corrosion resistance, which can be used in a facility for heat treatment of methionine dimer.

An object of the present invention is to perform stable operation of a process for producing methionine by lengthening the life of a facility for recovery through a heat treatment in a process for producing methionine, which comprises hydrolyzing 5-(β-methylmercaptoethyl)hydantoin using at least one potassium compound selected from potassium carbonate, potassium bicarbonate and potassium hydroxide, crystallizing the hydrolyzate under pressure of carbonic acid gas thereby separating and collecting methionine, and heat treating at least a portion of the resulting filtrate thereby recovering methionine dimer contained in the filtrate as methionine.

The present invention provides a process for producing methionine, which comprises hydrolyzing 5-(β-methylmercaptoethyl)hydantoin using at least one potassium compound selected from potassium carbonate, potassium bicarbonate and potassium hydroxide, crystallizing the hydrolyzate under pressure of carbonic acid gas thereby separating and collecting methionine, and heat treating at least a portion of the resulting filtrate thereby recovering methionine dimer contained in the filtrate as methionine, the process being characterized by using, as a material of an equipment of a facility for recovery through a heat treatment, a ferritic stainless steel containing 17.0 to 32.0% by weight of Cr element, 1.0 to 3.0% by weight of Mo element and 0.6% by weight or less of Ni element, or zirconium.

According to the present invention, it becomes possible to stably produce methionine because a facility for recovery through a heat treatment of methionine dimer is less likely to be corroded in case of producing methionine from 5-(β-methylmercaptoethyl)hydantoin, and thus the present invention is of great industrial value.

In the present invention, an amount of potassium compound used in case of hydrolyzing (β-methylmercaptoethyl)hydantoin is commonly from 2 to 10 mols per mol of hydantoin in terms of an amount of potassium ions when the entire potassium compound is ionized. The hydrolysis temperature is commonly from about 120 to 220°C, and preferably from 160 to 190°C. In the present invention, after the hydrolysis, carbonic acid gas is supplied into a reactor and methionine is crystallized under pressure of carbonic acid gas. The precipitated methionine is separated by a solid-liquid separation method.
After methionine was separated by filtration, the resulting filtrate is commonly circulated so as to hydrolyze 5-(β-methylmercaptoethyl) hydantoin. When the filtrate is circulated for a long period, impurities and a decomposition product are accumulated in the filtrate, resulting in low purity of precipitated methionine. Therefore, so-called partial purging capable of removing the filtrate out of the system at a given rate is preferably conducted so as to avoid an increase of an amount of impurities and coloring components in the filtrate. Recovery of methionine from the filtrate in the present invention particularly exerts the effect for the partial purged filtrate, and also can be applied to the circulating filtrate.
The amount of the filtrate to be partially purged varies depending on the amount of impurities and coloring components in the circulating filtrate, but is commonly from about 5 to 20% based on the filtrate obtained by solid-liquid separation of methionine.

The heat treatment of the filtrate is performed as it is or after concentrating the filtrate. Commonly, the filtrate before the heat treatment contains about 30 to 60 g/liter of methionine and about 5 to 25 g/liter of methionine dimer.

The heat treatment temperature of the filtrate varies depending on the concentration of the methionine dimer contained, but is commonly within a range from about 150 to 200°C, and preferably from about 170 to 190°C. In case of low temperature, a hydrolysis rate of the methionine dimer is low. In case of high temperature, hydrolysis of the methionine dimer quickly proceeds, but there arises a problem such as heat deterioration of methionine.

In the present invention, methionine is recovered from the filtrate after subjecting to the heat treatment. The recovery may be performed by a known process such as process described in U.S. Patent No. 5,945,563. Specifically, the process includes a process which comprises adding water soluble solvent with or without partially concentrating the filtrate after subjecting to the heat treatment, and crystallizing methionine and potassium bicarbonate under pressure of carbonic acid gas, followed by separation and further recovery. Examples of the water soluble solvent to be added include alcohol such as isopropyl alcohol or methanol, and acetone.

The amount of the water soluble solvent to be used is commonly from about 0.2 to 2 parts by weight based on 1 part by weight of the filtrate. When the amount is too small, the recovery ratio of methionine and potassium bicarbonate decreases. Even when the amount is too large, the recovery ratio is not remarkably improved.

The pressure of the carbonic acid gas to be used is commonly from about 0.5 to 20 kg/cm$^2$G, and preferably from 2 to 6 kg/cm$^2$G, in terms of a gauge pressure. When the pressure is too low, the recovery ratio of methionine and potassium bicarbonate becomes insufficient regardless of the amount of the water soluble solvent to be used. Even when the pressure is too high, the recovery ratio is not further improved.

The crystallization temperature is preferably a low temperature. The crystallization temperature is preferably from -10 to 40°C, more preferably from 0 to 20°C, and particularly preferably about 10°C when crystal precipitation is completed.

Before and/or after the filtrate is heat treated, the concentrating operation may be performed and the conditions of the concentrating operation are not specifically limited as long as heat deterioration of methionine does not occur, and various conditions can be basically employed. In view of energy efficiency and corrosion of concentrating facility, the temperature of the concentrating operation is preferably from about 50 to 160°C, and more preferably from about 50 to 140°C. The concentrating operation and the heat treating operation can also be carried out at the same time and, in that case, the concentrating operation is performed under the conditions of the heat treating operation. It is not preferable in view of energy efficiency and others to perform the concentrating operation under comparatively severe conditions of the heat treating operation.

It becomes possible to remove impurities and coloring components, which are present in the circulating system, out of the system while effectively recovering methionine and potassium bicarbonate in the filtrate by treating the partially purged filtrate as described above.

In the present invention, as the material of an equipment used in a facility for recovering as methionine through a heat treatment of methionine dimer contained in the filtrate, a ferritic stainless steel containing 17.0 to 32.0% by weight of Cr element, 0.75 to 3.0% by weight of Mo element and 0.6% by weight or less of Ni element, or zirconium is used. The facility for recovering as methionine through a heat treatment of methionine dimer includes, for example, reactor for heat treatment, heat exchanger, concentrator, pipe arrangement, valve plug, and instrumentation facility, and these facilities are composed of the above ferritic stainless steel or zirconium, or these facilities are lined.

The Cr element in the ferritic stainless steel used in the present invention is an alloying element which is indispensable to improve the corrosion resistance. When the amount of the Cr element is too small, good corrosion resistance cannot be maintained. On the other hand, when the amount is too large, the resulting steel is likely to become brittle. Since the Ni element deteriorates corrosion resistance of the stainless steel in the facility for recovery through a heat treatment, the amount of the Ni element is preferably as small as possible. When the amount of the Mo element to be contained is within the above range, good corrosion resistance is exhibited in the facility for recovery through a heat treatment.

The amount of Ni element is preferably 0.5% by weight or less. The amount of Mo element is preferably 1.0% by weight or more, and preferably 2.5% by weight or less.

The existence of the other elements, which are not defined in the ferritic stainless steel used in the present invention, is not restricted, as long as corrosion resistance is not greatly deteriorated when used as the material of an equipment of a facility for recovery through a heat treatment. Examples of the other elements include C element, Si element, Mn element, P element, S element and N element. The balance is Fe.

The ferritic stainless steel used in the present invention is not specifically limited as long as it contains the above chemical components. Among commercially available ferritic stainless steels, SUS444, SUS445J2, SUS447J1 and SUSXM27 correspond to the above composition and the use of them is economical.

|  | C | Si | Mn | P | S | Cr | Mn | N | Ni |
|---|---|---|---|---|---|---|---|---|---|
| SUS444 | ≤0.025 | ≤1.00 | ≤1.00 | ≤0.040 | ≤0.030 | 17.0~20.00 | 1.75~2.50 | ≤0.025 | ≤0.60 |
| SUS445J2 | ≤0.025 | ≤1.00 | ≤1.00 | ≤0.040 | ≤0.030 | 21.00~24.00 | 1.50~2.50 | ≤0.025 | ≤0.60 |
| SUS447J1 | ≤0.010 | ≤0.40 | ≤0.40 | ≤0.030 | ≤0.020 | 28.50~32.00 | 1.50~2.50 | ≤0.015 | ≤0.50 |
| SUSXM27 | ≤0.010 | ≤0.40 | ≤0.40 | ≤0.030 | ≤0.020 | 25.00~27.50 | 0.75~1.50 | ≤0.015 | ≤0.50 |

Zirconium used in the present invention is zirconium for industrial use and commonly has purity of 95.5% or more, and preferably 99.2% or more. It is difficult to separate zirconium from hafnium because of their very similar chemical properties. Zirconium for industrial use commonly contains about 2% hafnium, but exerts less influence on corrosion resistance. As used herein, purity means the value Zr (+Hf) containing a small amount of hafnium. Zirconium contains hafnium in an amount of 4.5% at most (ASTM B551).

Examples

While the present invention will now be described by way of Examples, it should be understood that these are exemplary of the present invention and are not to be considered as limiting.
The content of chemical components of the specimen in the Examples is measured using a fluorescent X-ray spectrometer.

Example 1, Comparative Example 1

Each specimen shown in Table 1 was inserted into the liquid phase portion and the vapor phase portion of a 1 liter autoclave and 600 ml of methionine crystallization filtrate containing 4% by weight of methionine, 2% by weight of methionine dimer and 12% by weight (in terms of potassium ions) of potassium compound was charged in the autoclave, and then a corrosion test was carried out while maintaining at a temperature of 180°C for 8 hours.
The weights of the specimen before and after the test were measured, and a corrosion rate (mm/year) was determined by the following equation. The results are shown in Table 1.
Corrosion rate = {(W1 - W2)/(d x S)}/testing time $\times$ 24 $\times$ 365

W1:   Weight of specimen before test (g)
W2:   Weight of specimen after test (g)
d:    Density of specimen (g/mm$^3$)
S:    Surface area of specimen (mm$^2$)

Table 1

| | | Chemical composition (wt%) | | | | Corrosion rate (mm/year) | |
|---|---|---|---|---|---|---|---|
| | | Cr | Ni | Mo | Zr (+Hf) | Liquid phase | Vapor phase |
| Example 1 | Zirconium | - | - | - | 99.6 | 0.01 | 0.01 |
| Comparative Example 1 | SUS329J4L | 25.09 | 7.32 | 3.18 | - | 3.02 | 0.01 |

Example 2, Comparative Example 2

Each specimen shown in Table 2 was inserted into the liquid phase portion of an autoclave and methionine crystallization filtrate containing 4% by weight of methionine, 2% by weight of methionine dimer and 12% by weight (in terms of potassium ions) of potassium compound was continuously charged in the autoclave at a rate of 4.7 liter/hour, and then a corrosion test was carried out while maintaining at a temperature of 178 to 185°C for 9 days.
The weights of the specimen before and after the test were measured, and a corrosion rate (mm/year) was determined. W1, W2, d and S are as defined in Example 1. The results are shown in Table 2.
$$\text{Corrosion rate} = \{(W1 - W2)/(d \times S)\}/\text{testing day} \times 365$$

Table 2

| | | Chemical composition (wt%) | | | | Corrosion rate (mm/year) |
|---|---|---|---|---|---|---|
| | | Cr | Ni | Mo | Zr (+Hf) | Liquid phase |
| Example 2 | SUS447J1 | 29.8 | 0.18 | 1.94 | - | 0.03 |
| | Zirconium | - | - | - | 99.6 | 0.01 |

(continued)

| | | Chemical composition (wt%) | | | | Corrosion rate (mm/year) |
|---|---|---|---|---|---|---|
| | | Cr | Ni | Mo | Zr (+Hf) | Liquid phase |
| Comparative Example 2 | SUS329J4L | 25.09 | 7.32 | 3.18 | - | 2.41 |

As described above, a corrosion rate of SUS447J1 as a ferritic stainless steel containing 17.0 to 32.0% by weight of Cr element, 1.0 to 3.0% by weight of Mo element and 0.6% by weight or less of Ni element, and that of zirconium are respectively 0.1 mm/year or less and, when they are used as the material of an equipment of a facility for recovering as methionine through a heat treatment of methionine dimer, the facility is less likely to be corroded, and thus making it possible to stably produce methionine.

**Claims**

1.  A process for producing methionine, which comprises hydrolyzing 5-(β-methylmercaptoethyl)hydantoin using at least one potassium compound selected from potassium carbonate, potassium bicarbonate and potassium hydroxide, crystallizing the hydrolyzate under pressure of carbonic acid gas thereby separating and collecting methionine, and heat treating at least a portion of the resulting filtrate thereby recovering methionine dimer contained in the filtrate as methionine, the process being **characterized by** using, as a material of an equipment of a facility for recovery through a heat treatment, a ferritic stainless steel containing 17.0 to 32.0% by weight of Cr element, 0.75 to 3.0% by weight of Mo element and 0.6% by weight or less of Ni element, or zirconium.

2.  The process for producing methionine according to claim 1, wherein the ferritic stainless steel is SUS444, SUS445J2, SUS447J1 or SUSXM27.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5945563 A